# EUROPÄISCHE PATENTSCHRIFT

(11) **EP 1 037 670 B1**
(45) Veröffentlichungstag und Bekanntmachung des Hinweises auf die Patenterteilung: **05.11.2003**
(21) Anmeldenummer: 98966568.2
(22) Anmeldetag: 14.12.1998
(51) Int. Cl.: A61K 48/00, A61P 35/00, A61P 25/28

(54) **MITTEL ZUR GENTHERAPIE VON TUMORERKRANKUNGEN, NEURODEGENERATIVEN, HERZKREISLAUF- UND AUTOIMMUNERKRANKUNGEN**
AGENT FOR GENE THERAPY OF TUMOR, NEURODEGENERATIVE, CARDIOVASCULAR AND AUTOIMMUNE DISEASES
AGENT POUR LA THERAPIE GENIQUE DE TUMEURS, MALADIES NEURODEGENERATIVES, CARDIOVASCULAIRES ET AUTO-IMMUNES

(30) Priorität: 12.12.1997 DE 19756309
(43) Veröffentlichungstag der Anmeldung: 27.09.2000
(73) Patentinhaber: MAX-DELBRÜCK-CENTRUM FÜR MOLEKULARE MEDIZIN, 13125 Berlin (DE)
(72) Erfinder: RESZKA, Regina, D-16341 Schwanebeck (DE); BERNDT, Antje, D-16341 Zepernick (DE)
(74) Vertreter: Baumbach, Friedrich, Dr.
(86) Internationale Anmeldenummer: DE9803763
(87) Internationale Veröffentlichungsnummer: WO99030741

(56) Entgegenhaltungen:
- EP-A- 0 132 142
- WO-A-95/12392
- WO-A-96/20698
- WO-A-98/47532
- DE-A- 4 341 478
- PAUSER S ET AL: "Evaluation of efficient chemoembolization mixtures by magnetic resonance imaging therapy monitoring: an experimental study on the VX2 tumor in the rabbit liver." CANCER RESEARCH, (1996 APR 15) 56 (8) 1863-7. JOURNAL CODE: CNF. ISSN: 0008-5472., XP002106971 United States
- DATABASE MEDLINE FILE SERVER STN KARLSRUHE ZUSAMMENFASSUNG 90358589, TAKEKOSHI H: "Chemoembolization combined with hepatic arterial induction of endogenous TNF and anticancer agents for hepatocellular carcinoma--a case report." XP002106973 & GAN TO KAGAKU RYOHO [JAPANESE JOURNAL OF CANCER AND CHEMOTHERAPY], (1990 AUG) 17 (8 PT 2) 1744-7. JOURNAL CODE: 6T8. ISSN: 0385-0684.,
- RESZKA, R. C (1) ET AL: "A new drug carrier application system for an efficient targeted treatment of liver metastases." JOURNAL OF LIPOSOME RESEARCH, (FEB., 1998) VOL. 8, NO. 1, PP. 98. MEETING INFO.: SIXTH LIPOSOME RESEARCH DAYS CONFERENCE LES EMBIEZ, FRANCE MAY 28-31, 1998 ISSN: 0898-2104., XP002106972

## Beschreibung

Die Erfindung betrifft Mittel zur Gentherapie von Tumorerkrankungen, neurodegenerativen, Herzkreislauf- und Autoimmunerkrankungen. Anwendungsgebiete der Erfindung sind die Medizin und die pharmazeutische Industrie.

Dickdarm- und Mastdarmtumoren sind nach den Bronchialkarzinomen bei Männern und Mammatumoren bei Frauen die häufigsten bösartigen (malignen) Tumoren in Deutschland. Die wesentliche therapeutische Maßnahme besteht bei diesen Patienten in der radikalen Resektion des tumortragenden Darmabschnitts. Die Hauptsache für die hohe Mortalität bei kolorektalen Karzinomen ist aber die Metastasierung (a. 1. Stelle Lebermetastasierung). Wenn keine metastasenchirurgische Behandlung mehr möglich ist, bleibt in aller Regel eine intensive Chemotherapie als letztes Mittel der Wahl.

Trotz intensivster Erforschung neuer potenter Chemotherapeutika ist die Behandlung nicht resektabler Tumoren, insbesondere von Lebermetastasen, weiterhin problematisch, denn kolorektale Karzinome verfügen über eine geringe Zellproliferationsrate, Tumorheterogenität und Medikamentenresistenz. Ein anderer Grund für das häufige Versagen der Chemotherapie liegt darin, daß die gegenwärtig verfügbaren Zytostatika weder selektiv an bestimmten Stoffwechselwegen von Tumoren angreifen, noch ausschließlich gegen Tumorzellen gerichtet sind. Folglich geht der Einsatz von Zytostatika mit vielen schweren Nebenwirkungen einher.

Hier bietet der Einsatz von Liposomen und Polymeren die Möglichkeit, modifiziert in die pharmakologischen Eigenschaften der Chemotherapeutika einzugreifen.

Trotz jahrzehntelanger intensiver Bemühungen, Patienten mit inoperablen Tumoren mit Hilfe der Chemotherapie zu heilen, sind die Fortschritte als gering zu bezeichnen. Mit Ausnahme einiger weniger Erkrankungen (z.B: akute lymphatische Leukämie) ist eine vollständige Heilung des Patienten durch alleinige chemotherapeutische Maßnahmen nicht möglich. In vielen Fällen ist auch keine signifikante Erhöhung der Lebenserwartung festzustellen. Dies liegt einerseits an der geringen Tumorspezifität vieler Chemotherapeutika und andererseits an der relativ hohen Toxizität dieser Substanzen. In der Folge ist häufig, trotz massiver Nebenwirkungen, kein ausreichender Wirkspiegel im Tumor zu beobachten. In 28 verschiedenen Studien wurden insgesamt 663 Patienten mit 22 verschiedenen Substanzen behandelt. Nur 6 Patienten zeigten eine komplette Tumorregression, 63 eine temporäre, die jedoch in den meisten Fällen nicht zu einer signifikanten Prolongation der Überlebenszeit führte. Diese Studien belegen eindeutig die Notwendigkeit, bestehende Therapiekonzepte zu verbessern und gegebenenfalls neue Ansätze zu entwickeln.

Liposomen werden seit 23 Jahren u.a. wegen ihrer Ähnlichkeit zu Zellmembranen als multifunktionelles Träger- und Transportsystem für biologisch aktive Substanzen, einschließlich pround eukaryontischer Gene untersucht. (Kim S., Drugs, 1993, 46:618-638). Sie lassen sich als geschlossene mikroskopische Strukturen charakterisieren, die aus konzentrisch geordneten Lipiddoppelschichten bestehen, die ihrerseits wäßrige Kompartimente voneinander abtrennen. Besonders umfangreich sind die Arbeiten zur liposomalen Verkapselung von Arzneimitteln. Hier bieten die Liposomen im Vergleich zu anderen Trägersystemen Vorteile, die sich u.a. auch für die Verkapselung von DNA-Konstrukten ausnutzen lassen.
- Wählbarkeit der Zusammensetzung, Ladung, Größe und Stabilität je nach Fragestellung.
- Möglichkeit des vollständigen biologischen Abbaus.
- kaum vorhandene immunologische und toxische Reaktionen.
- Häufig veränderte Pharmakokinetik der liposomal verkapselten Substanz.
- Veränderte Organverteilung und Tropie zu bestimmten Organen.
- Möglichkeiten für verschiedene Methoden des Targetings (Antikörper, Lectine)

Liposomen können auch als Gentransfer-Systeme verwendet werden.

In der Mehrzahl der tierexperimentellen Modelle wurde der Gentransfer jedoch ex vivo durchgeführt. Die hierdurch gewonnenen Erkenntnisse über eine durch die genmodifizierten Tumorzellen induzierte tumorspezifische Immunantwort hat zu der Strategie einer 'Vakzinierung' mit Zytokin-Gentransfizierten Tumorzellen geführt. Eine In-vivo-Gentransfer-Strategie wurde im Rahmen einer RAC-genehmigten Studie im University of Michigan Medical Center, Ann Arbor angewandt. Durch die direkte Injektion von Liposomen/Plasmid-DNA-Komplexen im Tumorgewebe soll der Transfer eines MHC-Klasse-I (HLA-B7)-Gens in Tumorzellen erreicht werden, um dadurch eine Immunreaktion zu stimulieren. Andere Gentransfersysteme benutzen Suizid-Gene, um Tumorzellen für chemotherapeutische Substanzen sensitiv zu machen. Dabei sind verschiedene Gene getestet worden, die ein selektives Abtöten der exprimierten Zellen verursachen können.

Ein einfaches und nach den ersten klinischen Daten auch wirkungsvolles System wurde von K. Culver entwickelt (Culver K.W. et al., Science 1992, 256:1550) und bereits in klinischen Studien angewandt. Die Strategie basiert auf dem Transfer des Herpes-Simplex-thymidinkinase (HSV-tk)-Gens in Tumorzellen mittels eines retroviralen Vektors. HSV-tk-transfizierte Zellen werden für die Anti-Virus-Substanz Ganciclovir sensitiv. Ganciclovir wird durch HSV-tk in einem Nukleotid-ähnlichen Precursor, der nach weiterer Phosphorylierung in die DNA teilender Zellen inkorpiert wird und zu einem Stopp der DNA-Synthese und zum Zelltod führt.

Caruso et al. (Proc. natl. Acad. Sci., 1993, 90:7024-7028) konnten durch Adaptation der Culver-Suicidstrategie an das Lebermetastasemodell nach Injektion von HSV-tk-Vektorproduzierenden Zellen eine Regression von etablierten, makroskopisch sichtbaren Lebermetastasen nachweisen. Nachteilig für diesen Ansatz der Tumortherapie mit viralen Vektoren ist aber, daß auf Grund der immunologischen Abwehrmechanismen des Organismus, nur eine einmalige Gabe des Gen-Vektor-Konstruktes möglich ist. Für einen liposomalen Vektor ist eine mehrmalige systemische Verarbeitung möglich.

Auch für die Behandlung von relativ schwer zugänglichen Tumoren (z.B. multiple Lebermetastasen, Gehirntumore) ist die gezielte und sichere Applikation und Transfektion mit retro- bzw. adenoviralen Vektoren noch problematisch, ganz abgesehen von den auftretenden Behandlungsrisiken mit viralen Infektionen. Es soll möglichst wenig gesundes Gewebe zerstört und in Mitleidenschaft gezogen werden, bei möglichst hoher Transfereffizienz mit anschließender vollständiger Tumorregression.

Bisher wurde in vivo noch kein liposomal verpacktes Therapieoder Suizid-Gen in Lebermetastasen am CC531 Karzinom transfiziert.

Der Erfindung liegt die Aufgabe zugrunde, völlig neue Ansatzpunkte für eine lokoregionäre Therapie von Tumoren, insbesondere von Lebermetastasen, durch eine kombinierte Anwendung von Liposomen/Plasmid-DNA-Komplexen unterschiedlicher Zusammensetzung, Größe und Beladung zu liefern.

Die Aufgabe der Erfindung wird durch die in den Ansprüchen 1, 23 und 25 beschriebenen Merkmale gelöst; die Unteransprüche sind Vorzugsvarianten.

Kernpunkt der Erfindung ist ein pharmazeutisches Mittel, umfassend
- eine oder mehrere unverkapselte oder in PEG-, Immuno-, Immuno/PEG-, kationischen, ggf. Polymer-modifizierten, Liposomen verkapselte genetische Materialien,
- lyophilisierte oder abbaubare Stärkepartikel und/oder Gelatine und/oder Polymerpartikel, wie z.B. Nanopartikel und
- jod-, gadolinium-, magnetit- oder fluor-haltige Kontrastmittel.

Genetische Materialien sind bevorzugt DNA, RNA, Ribozyme, Antisense-Oligonukleotide und besonders bevorzugt Therapiegene, wie z.B. Suizidgene, Zytokingene, Chemokingene (MIP1α, MCP) Antiangiogenesegene, wie Vascular Endothial Growth Factor (VEGF), Apoptosegene, wie z.B. Apoptin, Natural born Killer (NbK), ggf. in Kombination mit Markergenen, wie z.B. Green Fluorescence Protein (GFP), Galactosidasegen (LacZ) unter ggf. induzierbaren, ggf. gewebespezifischen Promotoren.

Eine weitere Variante des Mittels besteht darin, daß es zusätzlich, die DNA dichter packende Proteine, wie Nuclear Capsid Protein (NCP 7), HMG und/oder synthetische Substanzen, wie z.B. Polyethylenimin, Poly-L-Lysin oder Protaminsulfat enthält.

Bevorzugte Suizidgene sind Herpes simplex Virus Thymidinkinasegen (HSVtk), Deaminasegen, NR/CB1954, Pyrin Nukleosid Phosphorylase und/oder die Zytokingene IL-2, IL-4, IL-6, IL-10, IL-12 und/oder IL-15.

Die Liposomen bestehen aus
a) einem natürlichen, halbsynthetischen oder vollsynthetischen Amphiphil
b) einem Steroid,
c) einer geladenen Lipidkomponente,
d) dem wasser- oder lipidlöslichen genetischen Material und/oder
e) einer Trägerflüssigkeit und ggf. zusätzlichen Hilfsstoffen.

Dabei sind die Mengenverhältnisse a:b:c bevorzugt im Molverhältnis 1:0,3:0,1 bis 1:1:0,1 oder bis 1:1:0,5 und c:d im Molverhältnis 2:1 bis 10:1.
Das natürliche, halbsynthetische oder vollsynthetische Amphiphil ist bevorzugt ein Lipid, Tensid, Emulgator, Polyethylenglykol (PEG) oder Lipid-PEG, wobei das Amphiphil eine Verbindung der allgemeinen Formel I ist 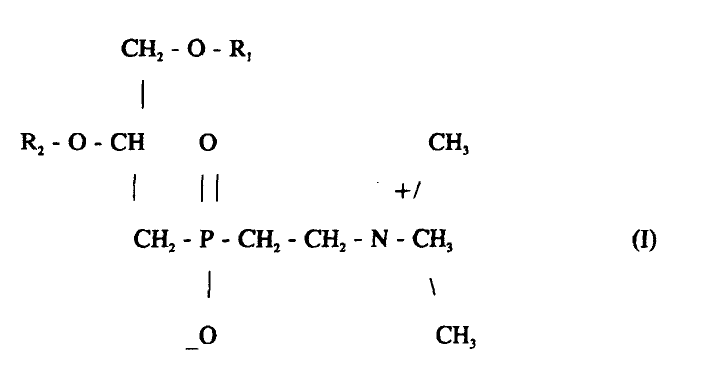 worin R₁ und R₂ = C₁₀ - C₂₀ - Alkanoyl, - Alkenoyl, - Alkyl, -Alkenyl bedeuten.

Das eingesetzte Steroid ist Cholesterol, Diethoxycholesterol oder Sitosterol.

Die geladene Lipidkomponente ist das Anion des Dicetylphosphats, der Palmitinsäure, der Stearinsäure, das Anion eines Phospholipids, wie z.B. Phosphatidylserin, Phosphatidsäure oder das Anion eines Sphingolipids, wie z.B. Sulfatid, oder Polyethylenglykol (PEG), wie z.B. MPEG-DSPE.

Eine bevorzugte Ausführungsform des Mittels besteht darin, daß die geladene Lipidkomponente fluoriert ist.

Weiterhin können als zusätzliche Hilfsstoffe Polymerpartikel in Form einer 25 %igen wäßrigen Lösung von Poloxamer eingesetzt werden.
Bevorzugt liegen die genetischen Materialien in
- SUV (Small unilamellar vesicles)-PEG-Liposomen,
- LUV (Large unilamellar vesicles)-PEG-Liposomen,
- REV (Reversed face evaporation vesicles)-PEG-Liposomen,
- MLV (Multilamellar vesicles)-PEG-Liposomen,
- Anti-Ki-67-Immun-PEG-Liposomen,
- Anti-CEA-PEG-Liposomen oder
- PEG DAC-Chol-Liposomen vor.

Die Stärkepartikel im Mittel liegen bevorzugt lyophilisiert in einer Größe von 40-90 um vor und befinden sich in physiologischer Kochsalzlösung in der Konzentration von 5 bis 70 mg/ml.
Besonders bevorzugt weisen die Stärkepartikel eine Korngröße von 60 bis 90 um auf.

Das erfindungsgemäße Mittel enthält als jodhaltiges Kontrastmittel ein- oder mehrfach jodierte Phenylderivate, darunter bevorzugt Iopromid, Ioxitalamat, Ioxaglat, Iopamidol, Iohexol, Iotralon, Metrizamid oder Ultravist.

Fluorierte Lipide kommen als Kontrastmittel ebenfalls in Betracht.

Eine sehr geeignete Ausführungsform ist ein Mittel, welches 30 bis 90 mg lyophilisierte oder abbaubare Stärkepartikel und 5 bis 100 mg verkapseltes oder unverkapseltes genetisches Material enthält.

Eine spezielle bevorzugte Ausführungsform des Mittels ist
dadurch gekennzeichnet, daß es
- Markergen LacZ und Suizidgen pUT HSVtk,
- verkapselt in MLV-PEG,
- als Stärkepartikel Spherex oder Gelfoam und
- ein fluoriertes Kontrastmittel enthält.

Die Mittel werden hergestellt, indem man 30 bis 90 mg lyophilisierte oder abbaubare Stärkepartikel und/oder Gelatine und/oder Polymerpartikel in 3 bis 6 ml Kontrastmittel löst und danach die therapeutisch notwendige Menge eines genetischen Materials zusetzt.

Bevorzugt wird die therapeutische Menge eines genetischen Materials und ggf. ein komplexierendes Agens in einem oder mehreren Lipiden gelöst und mit Stärkepartikeln und einem Kontrastmittel versetzt.

Die Verwendung der erfindungsgemäßen Mittel erfolgt zum Gentransfer und zur Gentherapie, insbesondere zur Therapie von Lebermetastasen, Tumoren der Lunge, Blase, Kopf und Hals, Urogenitalien, Lymphknoten, Mamma, bei Glioblastomen, Arthritis und Asthma.

Es kann gleichermaßen zur lokalen Gentherapie verwendet werden.

Speziell wird das Mittel verwendet
- zur intraarteriellen Therapie von Lebermetastasen, Pankreastumoren, Metastasen im kleinen Becken,
- zur Behandlung von neurodegenerativen und Autoimmunerkrankungen,
- bei Parkinsonscher, Alzheimer Krankheit und Multipler Sklerose,
- bei Diabetes Typ I,
- zur Begleitung von Transplantationen,
- zur Restenosebehandlung und
- bei Bluthochdruck.

Zusammengefaßt wird noch einmal festgestellt, daß für die Wirksamkeit der erfindungsgemäßen Liposomen/Plasmid-DNA-Komplexe folgende Zusammenhänge von entscheidender Bedeutung sind.
1. Die Anwendung der arteriellen Embolisationstherapie; (direkter Zugang zur Tumorversorgung (75-90%), hohe lokale Tumorkonzentration bei geringer systemischer Toxizität)
2. Die kombinierte Anwendung von DNA tragenden Liposomen mit dem Embolisat; (dreifaches Trägersystem)
3. Die Verwendung eines effektiven (starken) Promoters.

Als besonderer Vorteil ist hervorzuheben, daß der Organismus bei der Anwendung dieser Liposomen/Plasmid-DNA-Komplexe kaum eine immunologische Reaktion zeigt (wiederholter Einsatz ist uneingeschränkt möglich), es konnte darüber hinaus keine Toxizität festgestellt werden.

Die Erfindung soll nachfolgend durch Ausführungsbeispiele erläutert werden.

### Methode:

In vivo Untersuchung zur Behandlung von Lebermetastasen im Tiermodell.
Es werden multilamellare Polyethylenglykol-Liposomen (MLV-PEG-Liposomen) mit DNA (Suizidgen) beladen und zusammen mit dem Drug Carrier Embolisation System (DCES) intraarteriell zur Behandlung von Lebermetastasen der Ratte eingesetzt.

### Tiere:

Für die tierexperimentelle Untersuchungen werden männliche Wag/Rij Ratten verwendet (Zuchtbetrieb: Harlan Winkelmann GmbH, Gartenstrasse 27, D-33178 Borchen). Das durchschnittliche Körpergewicht (KGW) der Tiere beträgt 250-280g. Die Tierhaltung entspricht den Richtlinien des Tierschutzgesetzes, d.h. die spezifisch pathogen freien (SPF) Ratten werden unter standardisierten Umweltbedingungen im transgenen Trakt des Tierlaboratoriums im Max-Delbrück-Centrum, Berlin-Buch gehalten. Im Tierstall beträgt die Temperatur 22°C (±1°C), die relativen Luftfeuchtigkeit liegt bei 50% (±10%) und der Hell-Dunkel-Rhythmus (6.00-18.00Uhr) wechselt alle 12h. In einem standardisierten Käfig (Typ 3) mit 810cm² Bodengrundfläche und einer Höhe von 19cm werden die Ratten zu je 2 Tieren auf einer Einstreu aus Sägespäne gehalten (autoklaviert bei 121°C für 12min). Als Standardfutter erhalten die Tiere Versuchstierdiät für Ratten der Firma Sniff sowie Wasser ad libitum.

### Zellkultur:

Die CC531 Zellinie wird in RPMI 1640 Medium kultiviert. Das Medium enthält 10% fötales Kälberserum, 100U/ml Penizillin G, 100µg/ml Streptomyzinsulfat und 0,25µg/ml Amphoterizin. Die Zellen werden inkubiert im Zellinkubator bei 5% CO₂, 95% Luftfeuchtigkeit und bei 37°C.

### Herstellung der Liposomen:

Hydrogenisiertes Soja-phosphatidylcholin, Cholesterol, Polyethylenglykol wird im molaren Verhältnis von 1:1:0,1 in Chloroform gelöst. Im Rotationsverdampfer wird aus dieser Lösung Chloroform vollständig abgedampft und ein Lipidfilm entsteht. Der Lipidfilm wird mit Aqua dest. über 12h unter Schütteln inkubiert. Während des Schüttelns bilden sich MLV-PEG-Liposomen. Für die Herstellung der Liposomen/DNA-Komplexe wird in die Aqua dest. Phase die gewünschte DNA Konzentration hinzugefügt und wie beschrieben mit dem Lipidfilm geschüttelt. Dabei lagert sich die DNA zwischen die sich bildenden Lipiddoppelschichten.

Verwendete DNA:
- Reportergen pUT 651 kodiert das E. coli lacZ Gen (β-Galactosidase) CMV-Promoter
- Suizidgen pUT 649 kodiert das Herpes simplex Virus-Thymidin Kinase CMV-Promoter
- Suizidgen pBS-CEA-tk kodiert das Herpes simplex Virus-Thymidin Kinase CEA-Promoter

### Vorbereitung und Narkose der Versuchstiere:

Für alle tierexperimentellen Arbeiten werden die Ratten narkotisiert. Dafür werden sie ca. ½-1min in einen Ethertopf gesetzt und bei einsetzender Immobilisation heraugenommen. Die Injektionsnarkotika werden über eine 1ml Mischspritze i.m. (Oberschenkelmuskulatur) appliziert. Erst nach vollständigem Einsetzen der anästhetischen und analgetischen Wirkung (nach ca. 10min) wird mit der weiteren Behandlung fortgefahren.

| **Narkotika** | **Dosis** |
|---|---|
| 1. Xylazin (Rompun®, 2%, Bayer AG, Leverkusen) | 12mg/kg KGW |
| 2. Ketaminhydrochlorid (Ketanest®, 50mg/ml, PARKE-DAVIS GmbH, Berlin) | 80mg/kg KGW |

Für operative Eingriffe werden die Ratten am Abdomen geschoren (Schermaschine). Die ausgebundenen und fixierten Tiere werden an der rasierten Fläche gereinigt und mit 70% Ethanol desinfiziert.

### Zellaufbereitung der CC531 Zellen für die Tumorinokulation:

Aus der Zellkulturflasche wird das RPMI Medium abgesaugt und mit 3-4ml Trypsinlösung wird der Zellrasen kurz abgewaschen. Danach wird 1ml (25cm³ Zellkulturflasche) oder 1,5ml (75cm³ Zellkulturflasche) Trypsinlösung auf die Zellen gegeben und für 10min im Brutschrank inkubiert. Danach wird die Flasche herausgenommen und in 5ml (25cm³ Zellkulturflasche) bzw. 10ml (75cm³ Zellkulturflasche) RPMI Medium aufgenommen. Für die Auszählung der vitalen Zellen werden 50µl der Zellsuspension mit 50ml Trypanblau gefärbt (Lebend-Tod-Färbung). Tote Zellen färben sich auf Grund der erhöhten Zellpermeabilität blau an. Es werden in der Neubauer Zählkammer nur die vitalen Zellen gezählt und auf das gesamte Volumen umgerechnet.
Die Zellsuspension wird 2 mal bei 8000-1000Upm 3-5 min mitPhosphat gepufferte Saline (PBS) gewaschen und dann mit dem benötigten PBS-Volumen (3 x 10⁵/ 100µlPBS /Tier) aufgefüllt.

### Inokulation der CC531 Zellen:

Den narkotisierten Ratten wird in der Linea alba, caudal des Xyphoides (ca. 2cm lang) das Abdomen eröffnet. Anschließend wird der linke Leberlappen hervorgelagert. Dazu wird eine sterile, mit 0,9% igen NaCl-Lösung getränkte, Mullbinde (5cm x 5cm) verwendet, in dem der Leberlappen mit dem Mulltuch vorsichtig erfaßt und ca 3-4cm herausgezogen wird. Der vorgelagete Leberanteil wird auf der Bauchdecke auf feuchte Mullkompressen gelegt. Über eine subkapsulären Einstich in den linken Leberlappen (Kanüle: 27G x ¾", Nr.20, 0,4 x 20mm, TERMUO, Madrid) wird langsam die frisch aufbereitete Zellsuspension injiziert (3x10⁵ vitale Tumorzellen in 100µl PBS pro Tier). Beim Herausziehen der Kanüle wird mit einem Wattestäbchen der Einstichkanal abgedrückt, um das Abfließen der Zellsuspension zu verhindern. Mit einem Tropfen Gewebekleber (Histoacryl®, B. Braun Surgical GmbH, Melsungen) wird dann die Einstichsöffnung verschlossen und das Wattestächen kann entfernt werden. Der Leberlappen wird wieder vorsichtig in die Bauchhöhle zurückgelagert und die Bauchdecke wird zugenäht.
10-14 Tage nach der Tumorinokulation ist ein tastbarer (ca. 1cm³) Tumor in der Leber gewachsen.

### Intraarterielle Applikation der DNA/Liposomen-Komplexe:

Den narkotisierten Ratten wird parallel zu den Rippenbögen, caudal des Xyphoides, ca. 5-6cm lang, das Abdomen eröffnet. Mit Hilfe von angefeuchteten Mullkompressen werden die Leberanteile nach cranial und die Darmanteile nach caudal gedrängt, so daß die tieferen Regionen ohne Hindernis betrachtet werden können. Die weiteren Schritte erfolgen unter mikrochirurgischen Bedingungen. Unter Sicht des Mikroskopes 5-8 facher Vergrößerung werden die A. communis (A. hepatica), A. gastroduodenalis und A. hepatica propria freipräpariert.

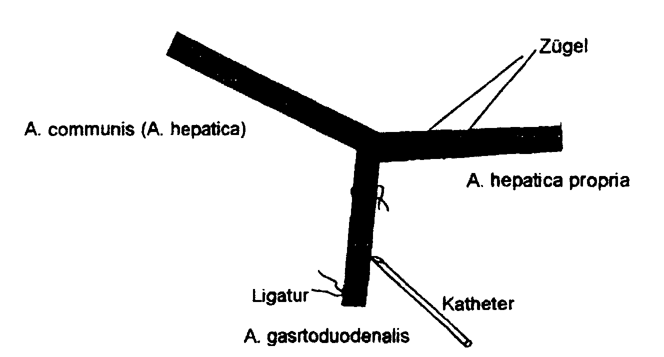

Um die A. hepatica propria wird mit einem Seidenfaden (5/0, 1 metric, Perma-Hand® Seide, geflochten, ETHICON, Norderstedt) ein Zügel angelegt und die A. gastroduodenalis wird nach distal ligiert. Nahe an dem Abgang der A. communis wird um die A. gastroduodenalis ein Faden mit gelockertem Knoten gelegt und anschließend proximal der Ligatur arteriotomiert. Ein Katheter wird vorsichtig über diese Öffnung in das Gefäß eingebracht und weiter nach intravasal vorgeschoben. Der so plazierte Katheterschlauch wird mit dem vorgelegten Knoten einmalig fixiert. Über eine Kanüle am anderen Ende des Schlauches wird Ringerlösung (B. Braun Melsungen AG, Meslungen) unter Sichtkontrolle injiziert (ca. 0,2ml), um das korrekte Abfließen der Flüssigkeit und den Kathetersitzes im Gefäß zu kontrollieren. Danach erfolgt die Applikation der Liposomen/DNA-Komlexe (mit DCES) im Wechsel von 50µl Liposomen und ½ min Blutfluß durch die A. communis (Lockerung des angezügelten Gefäßes). Nach vollständiger Injektion wird der Kathederschlauch nochmals mit Ringerlösung gespült (0,2-0,3ml) und aus dem Gefäß herausgezogen. Mit dem vorgelegten Knoten kann dann die A. gastroduodenalis proximal der Öffnung abgebunden werden. Danach werden die Mullkompressen entfernt und die Ratten werden weiter versorgt.

### Behandlung der Ratten mit Ganciclovir:

5 Tage nach der intraarteriellen Applikation der Liposomen/DNA-Komplexe erfolgt eine 14 tägige Behandlung der Ratten mit Ganciclovir-Natrium (Cymeven® i.v., Hoffmann-La Roche, Grenzach-Wyhlen). Dabei wird den Tieren 1 x täglich 100mg/kg KGW Ganciclovir intraperitoneal appliziert. Dafür werden die Ratten mit einer Hand im Nacken erfaßt und aus dem Käfig gehoben. Über eine 1ml Spritze mit einer sterilen Kanüle (27G x ¾", Nr.20, 0,4 x 20mm, TERMUO, Madrid) wird im Bereich der Mittellinie nach prüfender Aspiration in das Abdomen langsam injiziert.

### Tötung der Versuchstiere und Blutentnahme:

Am Tag der Tötung werden die Ratten narkotisiert und nach dem Einsetzen vollständiger Analgesie und Anästhesie intracardial (i.c.) mit einer 1ml Spritze (Omnifix®-F, Einmalspritze, B. Braun Melsungen AG, Melsungen) punktiert. Das einzelne Tier wird dabei in Rückenlage gebracht und auf der Höhe einer gedachten Verbindungslinie zwischen den beiden Ellenbogen so in den Thorax eingestochen, daß die Kanüle (25G x 5/8, 0,5 x 16mm) rechts des Sternums in kraniodorsaler Richtung geführt wird. Die Herzpunktion erfolgt bis zum Exitus des Tieres (ca. 4-6ml Vollblut).
Das gewonnene Blut wird für 1-2 Stunden bei Zimmertemperatur oder über Nacht bei 4°C stehen gelassen. Der entstandene Blutkuchen wird dann vorsichtig von der Röhrchenwand gelößt und bei max. 3000UpM 5min zentrifugiert. Danach wird vorsichtig der Überstand (Serum) abgehebert und eventuell nochmals zentrifugiert, um Erythrozytenbeimengungen zu beseitigen. Das so gewonnene Serum wird dann tiefgefroren und bei -20°C gelagert.

### Organentnahme und Aufbereitung:

Den toten Ratten wird das Abdomen und der Thorax geöffnet, und die benötigten Organe (Lebertumor, Leber, Niere, Bauchspeicheldrüse, Milz, Herz, Lunge, Lymphknoten (aus Darmgekröse und Achseln)) werden entnommen. Alle Organe werden mit 2-Methylbutan (Roth, Karlsruhe) als Kältevermittler bei -40°C tiefgefroren und bei -70°C gelagert.

### Bestimmung der Tumorgröße:

Die Tumorgröße wird nach der Formel V= a x b²/2 bestimmt, wobei a die größte und b die kleinste Tumorausdehnung bezeichnet. Die Tumordiameter werden bei jedem Tier zwei mal (Tag 10 und Tag 30) gemessen.

### Serumuntersuchung:

Die gewonnen Serumproben werden mit dem veterinärmedizinischen Analysegerät Vet Test 8008 (IDEXX GmbH, Wörrstadt, Deutschland) auf 16 verschiedene Blutparameter untersucht (photometrische Messung). Dabei handelte es sich um folgende Substanzen und Enzyme:

| Substrate | intrazelluläre Enzyme | exkretorische Enzyme |
|---|---|---|
| Albumin [g/dl] | Creatininkinase [mg/dl] | α-Amylase [IU] |
| Total Billirubin [mg/dl] | Aspartat-Aminotransferase [IU] | Lipase [IU] |
| Cholesterin [mg/dl] | Alanin-Aminotransferase [IUJ | |
| Creatinin [mg/dl] | Lactat-Dehydrogenase [IU] | |
| BUN (Harnstoff) [mg/dl] | Alkalische Phosphatase [IU] | |
| Glukose [mg/dl] | γ-Glutamyl-Transferase [IU] | |
| Ammoniak [mg/dl] | | |

### Herstellung der Gefrierschnitte:

Aus den tiefgefrorenen Organen (Leber, Lebertumor, Lunge, Niere Milz) werden am Kryotom (Kryostat von LEICA Instruments GmbH; Nußloch) 8-12µm dicke Gefrierschnitte angefertigt. Die Schnitte werden auf Poly-L-Lysin beschichtete Glasobjekträger aufgezogen und bei Raumtemperatur angetrocknet. Je nach weiterer Verwendung werden die Schnitte in 2% Paraformaldehydlösung bei 4°C 5min oder für 5min in eisgekühltem Aceton fixiert. Die Schnitte werden dann luftgetrocknet und bei -20°C gelagert oder gleich anschließend gefärbt.

### Histologische Auswertung

### Hämalaun-Eosin Färbung (Übersichtsfärbung) :

Für die histologischen Übersichtspräparate wird eine Doppelfärbung mit Hämalaun-Eosin angefertigt. Durch das Hämalaun nach MAYER werden basophile Zellstrukturen blau angefärbt (selektive blaue Kernfärbung). Für die Plasmafärbung wird mit dem Anilinfarbstoff Eosin Y rot gegengefärbt.

### X-Gal Färbung:

Zur Lokalisation der transfizierten Zellen und zur Auswertung der errreichten Transfereffizienz nach Applikation des liposomalen pUT651 Plasmides werden die angefertigten Gefrierschnitte folgendermaßen gefärbt. Die paraformaldehydfixierten Gefrierschnitte werden 2 mal für 5-10min in PBS bei Raumtemperatur gewaschen und sofort in die frisch vorbereitete Inkubationslösung*überführt und 4-24h bei 37°C inkubiert (Entwicklung der Blaufärbung). Anschließend werden die Schnitte kurz in Aqua dest. gespült und ca. 30s-1min in Eosin inkubiert. Die Schnitte werden dann entwässert über die aufsteigende Alkoholreihe bis zum Xylol und mit Eukitt® eingedeckelt.

| * X-Gal Inkubationslösung: | | |
|---|---|---|
| für 80ml = | 84ml | 1,1mM MgCl₂ (22,36mg in 100ml PBS(pH7,2) |
| | 6ml | 50mM K₃[Fe(CN)₆] (1,645g in 100ml H₂O) |
| | 6ml | 50mM K₄[Fe(CN)₆] (2,112g in 100ml H₂O) |
| | 2,1ml | 20mg/ml X-Gal in N,N-Dimethylformamid |

### Schematischer Versuchsablauf:

| | | |
|---|---|---|
| **Tag 0** | Tumorinokulation | 3 x 10⁵ vitale CC531 Zellen in 100µl PBS werden subkabsulär in die Leber gespritzt; |
| 10 - 14 Tage danach | | in der Leber ist ein 1cm³ großer, solider Tumor gewachsen; |
| **Tag 10** | intraarterielle OP | das liposomale DCES mit dem Suizidgen wird über die Leberarterie appliziert; |
| **Tag 15** | Ganciclovirgabe | Beginn mit der GCV Applikation (i.p.) 100mg/kg Körpergewicht 1 x täglich 14 Tage lang; |
| **Tag 30** | Tötung | unter intracardialem Blutentzug |

### Reportergentransfer:

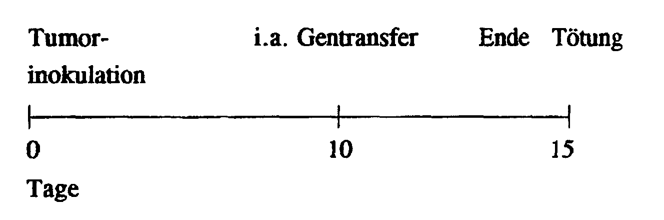

### Suicidgentransfer:

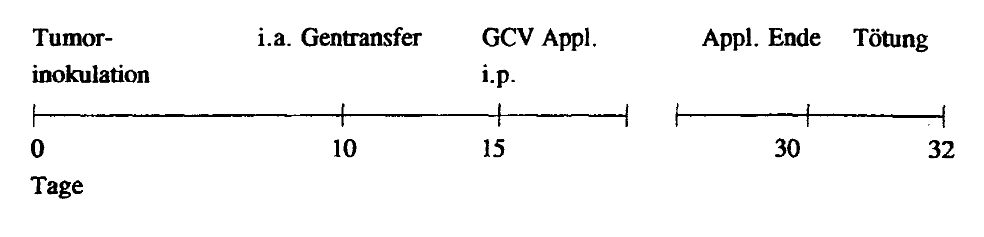

| Ergebnisse: | | | | |
|---|---|---|---|---|
| **Beispiel** | Plasmid | Liposomen | Embolisat | Verdünnung |
| **1** | 10µg pUT651 | 50µl MLV-PEG | 100µl Spherex® | 250µl Ringerlsg. |
| **2** | 10µg pUT649 | 50µl MLV-PEG | 100µl Spherex® | 250µl Ringerlsg. |
| **3** | 20µg pUT649 | 50µl MLV-PEG | 100µl Spherex® | 250µl Ringerlsg. |
| **4** | 10µg pBS-tk | 50µl MLV-PEG | 100µl Spherex® | 250µl Ringerlsg. |
| **Kontrolle** | ohne | ohne | ohne | 250µl Ringerlsg |

### Beispiel 1:

Den tumortragenden Ratten wird am Tag 10 mit dem DCES pUT651 (10µg) appliziert. 5 Tage danach ist die erwartete Transfektion des lacZ-Reportergenkonstruktes um den Tumor herum am stärksten und die Tiere werden getötet. Anschließend erfolgt die Organentnahme und die Aufarbeitung wie oben beschrieben. Es werden 12µm dünne Gefrierschnitte angefertigt, mit 2% Paraformaldehydlsg. fixiert, gewaschen und über Nacht in vorbereiteter X-Gal-Lösung, bei 37°C inkubiert. Der blaue β-Galaktosidase-Farbkomplex ist im Bereich des Tumorrandsaumes deutlich zu erkennen. Das bedeutet, daß mit diesem System in der empfindlichen Wachstumszone des Tumors gezielt DNA transfiziert werden kann. Alle anderen Organe sind in der Regel nicht betroffen.

### Beispiel 2:

Den tumortragenden Ratten wird am Tag 10 mit dem DCES pUT649 (10mg) appliziert. Während der Operation wird die Tumorgröße vermessen. 5 Tage danach ist die erwartete Transfektion des Suicidgenkonstruktes um den Tumor herum am stärksten und bei den Tieren wird die GCV Gabe begonnen (1 x täglich 100mg/kg KGW). Am Tag 30 werden die Tiere getötet. Anschließend erfolgt die Abmessung des Tumors, die Blut- und Organentnahme und die Aufarbeitung wie oben beschrieben. Es werden 12µm dünne Gefrierschnitte angefertigt, mit 2% Paraformaldehydlsg. fixiert, gewaschen und mit der Hämatoxylin/Eosin-Färbung angefärbt. Die Auswertung zeigte eine statistisch signifikante Verkleinerung der Lebermetastasen im Vergleich mit der Kontrollgruppe. Es ist keine vollständige Tumorregression feststellbar gewesen.
(siehe Diagramm im Anhang)

### Beispiel 3:

Den tumortragenden Ratten wird am Tag 10 mit dem DCES pUT649 (20mg) appliziert. Während der Operation wird die Tumorgröße vermessen. 5 Tage danach ist die erwartete Transfektion des Suicidgenkonstruktes um den Tumor herum am stärksten und bei den Tieren wird die GCV Gabe begonnen (1 x täglich 100mg/kg KGW). Am Tag 30 werden die Tiere getötet. Anschließend erfolgt die Abmessung des Tumors, die Blut- und Organentnahme und die Aufarbeitung wie oben beschrieben. Es werden 12µm dünne Gefrierschnitte angefertigt, mit 2% Paraformaldehydlsg. fixiert, gewaschen und mit der Hämatoxylin/Eosin-Färbung angefärbt. Die Auswertung zeigt eine statistisch signifikante Verkleinerung der Lebermetastasen im Vergleich mit der Kontrollgruppe. Es ist keine vollständige Tumorregression feststellbar.
(siehe Diagramm im Anhang)

### Beispiel 4:

Den tumortragenden Ratten wird am Tag 10 mit dem DCES pBS-tk (10mg) appliziert. Während der Operation wird die Tumorgröße vermessen. 5 Tage danach ist die erwartete Transfektion des Suicidgenkonstruktes um den Tumor herum am stärksten und bei den Tieren wird die GCV Gabe begonnen (1 x täglich 100mg/kg KGW). Am Tag 30 werden die Tiere getötet. Anschließend erfolgt die Abmessung des Tumors, die Blut- und Organentnahme und die Aufarbeitung wie oben beschrieben. Es werden 12µm dünne Gefrierschnitte angefertigt, mit 2% Paraformaldehydlsg. fixiert, gewaschen und mit der Hämatoxylin/Eosin-Färbung angefärbt. Die Auswertung zeigt, das auf Grund des schwächeren Promoters auch keine statistisch signifikante Verkleinerung der Lebermetastasen im Vergleich mit der Kontrollgruppe erreicht werden kann.
(siehe Diagramm im Anhang)

### Abkürzungen

- CEA: Promoter des Carcino-Embryonic Antigen
- CMV: Promoter des Cytomegalovirus
- DCES: Drug Carrier Embolisat System
- HSV-tk: Herpes Simplex Virus-thymidin kinase
- i.a.: intraarteriel
- i.p.: intraperitoneal
- KGW: Körpergewicht
- lacZ gen: Reportergen, codiert die β-Galaktosidase
- MLV-PEG: multilamellar vesicles-polyethylenglycol
- OP: Operation

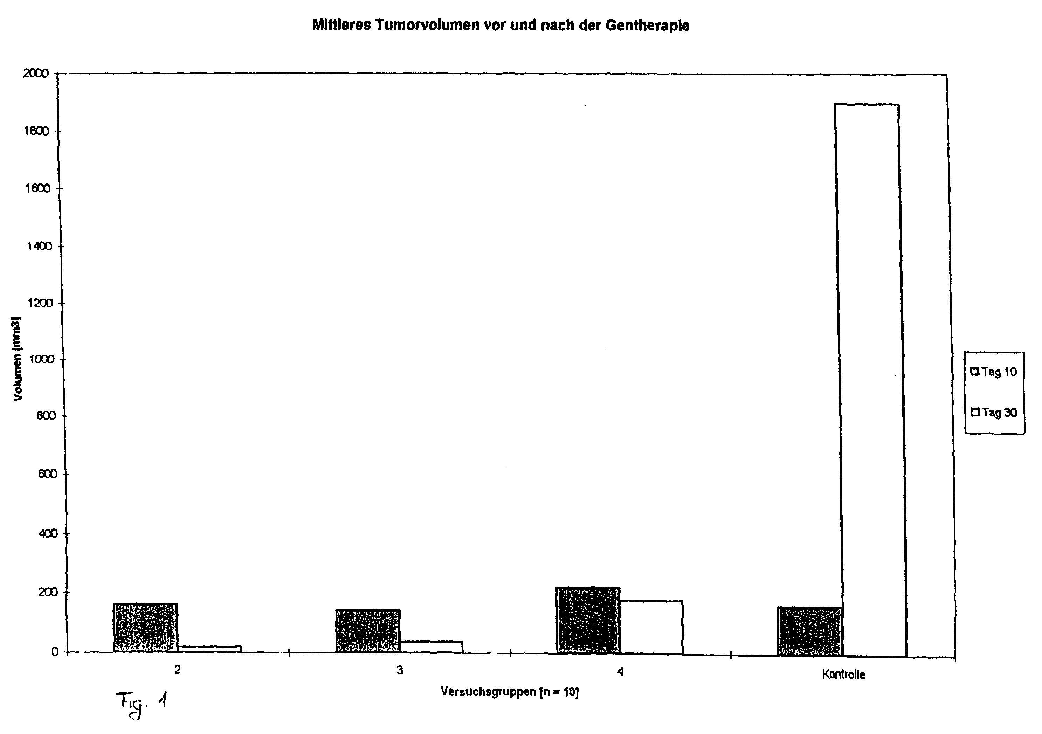

## Patentansprüche

1. Pharmazeutische Mittel umfassend
- eine oder mehrere unverkapselte oder in PEG-, Immuno-, Immuno/PEG-, kationischen, ggf. Polymer-modifizierten, Liposomen verkapselte genetische Materialien, insbesondere DNA, RNA, Ribozyme, Antisense-Oligonukleotide,
- lyophilisierte oder abbaubare Stärkepartikel und/oder Gelatine und/oder Polymerpartikel, wie z.B. Nanopartikel und
- jod-, gadolinium-, magnetit- oder Fluor-haltige Kontrastmittel.

2. Mittel nach Anspruch 1,
**dadurch gekennzeichnet, daß** es als genetische Materialien Therapiegene, wie z.B.
- Suizidgene, insbesondere die Suizidgene Herpes simplex Virus Thymidinkinasegen (HSVtk), Deaminasegen, NR/CB1954, Pyrin Nukleosid Phosphorylase und/oder die Zytokingene IL-2, IL-4, IL-6, IL-10, IL-12 und/oder IL-15,
- Zytokingene, insbesondere die Zytokingene IL-2, IL-4, IL-6, IL-10, IL-12 und/oder IL-15,
- Chemokingene (MIP1α, MCP),
- Antiangiogenesegene,
- Vascular Endothial Growth Factor (VEGF),
- Apoptosegene, wie z.B. Apoptin, Natural born Killer (NbK),
ggf. in Kombination mit Markergenen, wie z.B. Green Fluorescence Protein (GFP), Galactosidasegen (LacZ) unter ggf. induzierbaren, ggf. gewebespezifischen Promotoren,
ggf. in Kombination mit die DNA dichter packende Proteine wie Nuclear Capsid Protein (NCP 7), HMG und/oder synthetische Substanzen, wie z.B. Polyethylenimin, Poly-L-Lysin oder Protaminsulfat enthält.

3. Mittel nach einem der Ansprüche 1 oder 2
**dadurch gekennzeichnet, daß** die Liposomen aus einem
a) natürlichen, halbsynthetischen oder vollsynthetischen Amphiphil, insbesondere einem Lipid, Tensid, Emulgator, Polyethylenglykol (PEG) oder Lipid-PEG,
b) einem Steroid, insbesondere Cholesterol, Diethoxycholesterol oder Sitosterol
c) einer geladenen Lipidkomponente, insbesondere dem Anion des Dicetylphosphats, der Palmitinsäure, der Stearinsäure, dem Anion eines Phospholipids, wie z.B. Phosphatidylserin, Phosphatidsäure oder dem Anion eines Sphingolipids, wie *z.B.* Sulfatid, oder Polyethylenglykol (PEG), wie z.B. MPEG-DSPE, wobei die Lipidkomponente ggf. fluoriert ist,
d) dem wasser- oder lipidlöslichen genetischen Material und/oder
e) einer Trägerflüssigkeit und ggf. zusätzlichen Hilfsstoffen, insbesondere Polymerpartikel in Form einer 25 %igen wäßrigen Lösung von Poloxamer bestehen.

4. Mittel nach Anspruch 3,
**dadurch gekennzeichnet, daß** die Mengenverhältnisse a:b:c im Molverhältnis 1:0,3:0,1 bis 1:1:0,1 oder bis 1:1:0,5 und c:d im Molverhältnis 2:1 bis 10:1 sind.

5. Mittel nach Anspruch 3,
**dadurch gekennzeichnet, daß**
a) das Amphiphil eine Verbindung der allgemeinen Formel I ist 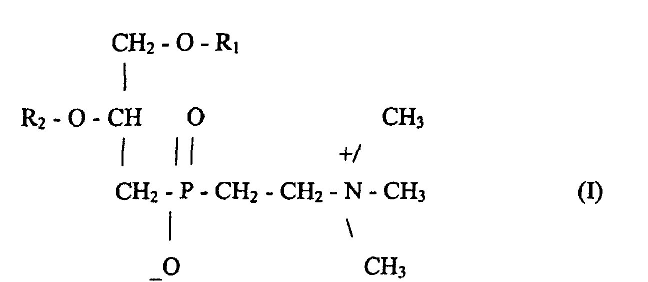
worin R₁ und R₂ = C₁₀ - C₂₀ - Alkanoyl, - Alkenoyl, - Alkyl, -Alkenyl bedeuten.

6. Mittel nach einem der Ansprüche 1 bis 5,
**dadurch gekennzeichnet, daß** die genetischen Materialien in
- SUV (Small unilamellar vesicles)-PEG- Liposomen,
- LUV (Large unilamellar vesicles)-PEG- Liposomen,
- REV (Reversed face evaporation vesicles)-PEG-Liposomen,
- MLV (Multilamellar vesicles)-PEG-Liposomen,
- Anti-Ki-67-Immun-PEG-Liposomen,
- Anti-CEA-PEG-Liposomen oder
- PEG DAC-Chol-Liposomen vorliegen.

7. Mittel nach einem der Ansprüche 1 bis 6,
**dadurch gekennzeichnet, daß**
- die Stärkepartikel lyophilisiert vorliegen in einer Größe von 40-90 µm und sie sich in physiologischer Kochsalzlösung in der Konzentration von 5 bis 70 mg/ml befinden,
- die Stärkepartikel eine Korngröße von 60 bis 90 µm aufweisen,
- absorbierbares Gelatinepuder enthalten ist,
- es als jodhaltiges Kontrastmittel ein- oder mehrfach jodierte Phenylderivate, insbesondere Iopromid, Ioxitalamat, Ioxaglat, Iopamidol, Iohexol, Iotralon, Metrizamid oder Ultravist enthält,
- es als Kontrastmittel fluorierte Lipide enthält und/oder
- daß es 30 bis 90 mg lyophilisierte oder abbaubare Stärkepartikel und 5 bis 100 mg verkapseltes oder unverkapseltes genetisches Material enthält.

8. Mittel nach einem der Ansprüche 1 bis 7,
**dadurch gekennzeichnet, daß** es
- Markergen LacZ und Suizidgen pUT HSVtk,
- verkapselt in MLV-PEG,
- Stärkepartikel Spherex oder Gelfoam und
- ein fluoriertes Kontrastmittel enthält.

9. Verfahren zur Herstellung eines Mittels gemäß einem der Ansprüche 1 bis 8, **dadurch gekennzeichnet, daß** man
- 30 bis 90 mg lyophilisierte oder abbaubare Stärkepartikel und/oder Gelatine und/oder Polymerpartikel in 3 bis 6 ml Kontrastmittel löst und danach die therapeutisch notwendige Menge eines genetischen Materials zusetzt oder
- die therapeutische Menge eines genetischen Materials und ggf. ein komplexierendes Agens in einem oder mehreren Lipiden löst und mit Stärkepartikeln und einem Kontrastmittel versetzt.

10. Verwendung von einem Mittel gemäß einem der Ansprüche 1 bis 9 zur Herstellung eines Arzneimittels
- zum Gentransfer und zur Gentherapie, insbesondere zur Therapie von Lebermetastasen, Tumoren der Lunge, Blase, Kopf und Hals, Urogenitalien, Lymphknoten, Mamma, bei Glioblastomen, Arthritis und Asthma,
- zur lokalen Gentherapie,
- zur intraarteriellen Therapie von Lebermetastasen,
- zur Behandlung von neurodegenerativen und Autoimmunerkrankungen,
- bei Parkinsonscher, Alzheimer Krankheit und Multipler Sklerose,
- bei Diabetes Typ I,
- zur Begleitung von Transplantationen,
- zur Restenosebehandlung und/oder
- bei Bluthochdruck.

## Claims

1. Pharmaceutical agents entailing
- one or more genetic material(s) unencapsulated or encapsulated in PEG, immuno, immuno/PEG, cationic, if need be polymer-modified, liposomes, in particular DNA, RNA, ribozymes, anti-sense oligonucleotides,
- lyophilised or decomposable starch particles and/or gelatine and/or polymer particles such as nano-particles and
- contrast agents containing iodine, gadolinium, magnetite or fluoride.

2. Agent according to Claim 1,
wherein it contains therapy genes as genetic materials, such as
- suicide genes, in particular the suicide gene Herpes simplex Virus thymidinkinase gene (HSVtk), deaminase gene, NR/CB1954, pyrine nucleoside phosphorylase and/or the cytokine genes IL-2, IL-4, IL-6, IL-10, IL-12 and/or IL-15,
- cytokine genes, in particular the cytokine genes IL-2, IL-4, IL-6, IL-10, IL-12 and/or IL-15,
- chemokine genes (MIP1α, MCP),
- antiangiogenesis genes,
- Vascular Endothelial Growth Factor (VEGF),
- Apoptosis genes, such as Apoptin, Natural born Killer (NbK),
if need be in combination with marker genes such as Green Fluorescence Protein (GFP), galactosidase gene (LacZ) under, if need be, inducible, possibly tissue-specific promoters,
if need be in combination with proteins packing the DNA more densely such as Nuclear Capsid Protein (NCP 7), HMG and/or synthetic substances such as polyethyleneimine, Poly-L-lysine or protamine sulphate.

3. Agent according to one of the claims 1 or 2,
wherein the liposomes comprise
a)a natural, semi-synthetic or totally synthetic amphiphile, in particular a lipid, tenside, emulsifier, polyethylene glycol (PEG) or lipid PEG,
b)a steroid, in particular cholesterol, diethoxy cholesterol or sitosterol,
c)a charged lipid component, in particular the anion of dicetyl phosphate, of palmitine acid, of stearic acid, the anion of a phospho-lipid such as phosphatidylserine, phosphatide acid or the anion of a sphingolipid, such as sulfatide, or polyethylene glycol (PEG), such as MPEG-DSPE, with the lipid component being fluorinated if need be,
d)the water or lipid-soluble genetic material and/or
e)a carrier fluid and, if need be, additional ancillary agents, in particular polymer particles in the form of a 25% watery solution of poloxamer.

4. Agent according to Claim 3,
wherein the quantity relationships a:b:c are in the mol relationship 1:0.3:0.1 to 1:1:0.1 or to 1:1:0.5 and c:d are in the mol relationship 2:1 to 10:1.

5. Agent according to Claim 3,
wherein
a) the amphiphile is a compound of the general formula I 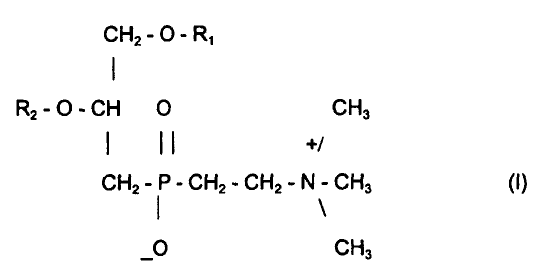 in which R₁ and R₂ mean C₁₀ - C₂₀ - alkanoyl, - alkenoyl, - alkyl, -alkenyl.

6. Agent according to one of the claims 1 to 5,
wherein the genetic materials exist in
- SUV (Small unilamellar vesicles)-PEG liposomes,
- LUV (Large unilamellar vesicles)-PEG liposomes,
- REV(Reversed face evaporation vesicles)-PEG liposomes,
- MLV (Multilamellar vesicles)-PEG liposomes,
- Anti-Ki-67-immuno-PEG liposomes,
- Anti-CEA-PEG liposomes or
- PEG DAC-Chol liposomes.

7. Agent according to one of the claims 1 to 6,
wherein
- the starch particles exist lyophilised with a size from 40-90 µm and are in a physiological saline solution in a concentration of 5 to 70 mg/ml,
- the starch particles manifest a grain size of 60 to 90 µm,
- absorbable gelatine powder is contained,
- it contains mono or polyiodised phenyl derivatives, in particular iopromide, ioxitalamate, ioxaglate, iopamidol, iohexol, iotralon, metrizamide or Ultravist as a contrast agent containing iodine,
- it contains fluorinated lipids as a contrast agent and/or
- it contains 30 to 90 mg of lyophilised or decomposable starch particles and 5 to 100 mg of encapsulated or unencapsulated genetic material.

8. Agent according to one of the claims 1 to 7,
wherein it contains
- marker gene LacZ and suicide gene pUT HSVtk,
- encapsulated in MLV-PEG,
- starch particles Spherex or Gelfoam and
- a fluorinated contrast agent.

9. Method for the production of an agent according to one of the Claims 1 to 8, wherein
- 30 to 90 mg of lyophilised or decomposable starch particles and/or gelatine and/or polymer particles are dissolved in 3 to 6 ml of contrast agent and then the therapeutically necessary quantity of a genetic material is added or
- the therapeutic quantity of a genetic material and, if need be, a complexing agent is dissolved in one or more lipids and starch particles and a contrast agent are added.

10. Use of an agent according to one of the claims 1 to 9 for the production of a medication
- for gene transfer and for gene therapy, in particular for therapy of hepatic metastases, tumours of the lung, bladder, head and neck, uro-genitals, lymph nodes, mamma, for glioblastomes, arthritis and asthma,
- for local gene therapy,
- for intra-arterial therapy of hepatic metastases,
- for treatment of neuro-degenerative and auto-immune diseases,
- for Morbus Parkinson, Alzheimer and multiple sclerosis,
- for type I diabetes,
- to accompany transplants,
- for restenosis treatment and/or
- for hypertonia.

## Revendications

1. Produit pharmaceutique comprenant
- une ou plusieurs matières génétiques non capsulées ou capsulées dans des liposomes PEG, immuno-, immuno/PEG-liposomes, cationiques, voire à polymérisation modifiée, en particulier A.D.N., A.R.N., ribozymes, oligonucléotides antisens,
- des particules d'amidon lyophilisées ou dégradables et/ou des gélatines et/ou des particules polymères, telles que les nanoparticules et
- une substance de contraste iodée, contentant du gadolinium, de la magnétite ou du fluor.

2. Produit selon la revendication 1,
**se caractérisant par le fait qu'**il contient en tant que matières génétiques des gènes thérapeutiques, tels que
- les gènes suicides, en particulier les gènes suicides de la kinase de thymidine du Virus simplex de l'Herpes (HSVtk), le gène de désaminase, le NR/CB1954, la phosphorylase de nucléoside de pyrine et/ou les gènes cytokines IL-2, IL-4, IL-6, IL-10, IL-12 et/ou IL-15,
- les gènes cytokines, en particulier les gènes cytokines IL-2, IL-4, IL-6, IL-10, IL-12 et/ou IL-15,
- les gènes chimiokines (MIP1α, MCP),
- les gènes antiangiogenèse,
- le facteur Vascular Endothial Growth Factor (VEGF),
- les gènes apoptoses, tels que l'apoptine, Natural born Killer (NbK),
le cas échéant, combinés avec des gènes marqueurs, tels que la Green Fluorescence Protein (GFP), les gènes de galactosidase (LacZ) sous des promoteurs évent. inductibles, évent. spécifiques au tissu,
le cas échéant, combinés avec des protéines condensant plus l'A.D.N. telles que la Nuclear Capsid Protein (NCP 7), HMG et/ou des substances synthétiques, telles que la Polyéthylénimine, la Poly-L-lysine ou le sulfate de protamine.

3. Produit selon l'une des revendications 1 ou 2,
**se caractérisant par le fait que** les liposomes se composent
a) d'un amphiphile naturel, semi-synthétique ou entièrement synthétique, en particulier d'un lipide, d'un tensioactif, d'un émulsionnant, d'un polyéthylène-glycol (PEG) ou d'un PEG lipide,
b) d'un stéroïde, en particulier le cholestérol, le diéthoxycholestérol ou le sitostérol,
c) d'une composante lipide chargée, en particulier de l'anion du phosphate dicétylique, de l'acide palmitique, de l'acide stéarique, de l'anion d'un phospholipide, tel que la phosphatidyl-sérine, l'acide phosphatidique ou de l'anion d'un sphingolipide, tel que la sulfatide, ou le polyéthylène-glycol (PEG), tel que MPEG-DSPE, la composante lipide étant le cas échéant fluorée,
d) de la matière génétique soluble dans l'eau ou dans le lipide et/ou
e) d'un liquide véhicule et le cas échéant d'agents auxiliaires, en particulier de particules polymères sous forme d'une solution aqueuse à 25 % de Poloxamer.

4. Produit selon la revendication 3,
**se caractérisant par le fait que** les rapports des ingrédients a :b :c sont en rapport molaire 1 :0,3 :0,1 à 1 :1 :0,1 ou à 1 :1 :0,5 et c :d en rapport molaire 2 :1 à 10 :1.

5. Produit selon la revendication 3,
**se caractérisant par le fait que**
a) l'amphiphile est un composé de la formule générale I 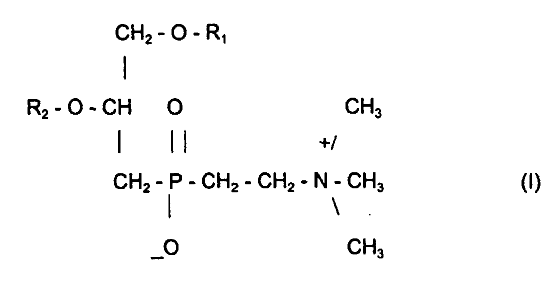 R₁ et R₂ étant = C₁₀ - C₂₀ - alcanoyle, - alcénoyle, -alkyle, -alcényle.

6. Produit selon l'une des revendications 1 à 5,
**se caractérisant par le fait que** les matières génétiques se trouvent dans
- des liposomes SUV (Small unilamellar vesicles)-PEG,
- des liposomes LUV (Large unilamellar vesicles)-PEG,
- des liposomes REV(Reversed face evaporation vesicles)-PEG,
- des liposomes MLV (Multilamellar vesicles)-PEG,
- des liposomes Anti-Ki-67-Immun-PEG,
- des liposomes Anti-CEA-PEG ou
- des PEG DAC-Chol liposomes.

7. Produit selon l'une des revendications 1 à 6,
**se caractérisant par le fait que**
- les particules d'amidon sous forme lyophilisée présentent un volume de 40-90 µm et se trouvent dans une solution de chlorure de sodium physiologique d'une concentration de 5 à 70 mg/ml.
- les particules d'amidon présentent une dimension de grain de 60 à 90 µm.
- qu'il contient une poudre de gélatine absorbable,
- qu'il contient en tant que substance de contraste iodée, des dérivés phényliques une ou plusieurs fois iodés, en particulier Iopromid, Ioxitalamat, Ioxaglat, Iopamidol, Iohexol, Iotralon, Metrizamid ou Ultravist,
- qu'il contient en tant que substance de contraste, des lipides fluorés et/ou
- qu'il contient des particules d'amidon lyophilisées ou dégradables de 30 à 90 mg et des matières génétiques capsulées ou non capsulées de 5 à 100 mg.

8. Produit selon l'une des revendications 1 à 7,
**se caractérisant par le fait qu'**il contient
- le gène marqueur LacZ et le gène suicide pUT HSVtk,
- capsulés dans MLV-PEG,
- la particule d'amidon Spherex ou Gelfoam et
- une substance de contraste fluorée.

9. Procédé permettant de fabriquer un produit conformément à l'une des revendications 1 à 8, **se caractérisant par le fait que** l'on
- dissout 30 à 90 mg de particules d'amidon lyophilisées ou dégradables et/ou de la gélatine et/ou des particules polymères dans 3 à 6 ml de substance de contraste et ensuite qu'on y ajoute la quantité thérapeutique nécessaire d'une matière génétique ou
- que l'on dissout la quantité thérapeutique d'une matière génétique et le cas échéant un agent complexant dans un ou plusieurs lipides et qu'on la mélange à des particules d'amidon et à une substance de contraste.

10. Emploi d'un produit conformément à l'une des revendications 1 à 9 pour la fabrication d'un médicament
- assurant un transfert génétique ou destiné à une thérapie génétique, en particulier pour le traitement de métastases du foie, de tumeurs de la langue, de la vessie, de la tête et du cou, de l'appareil génito-urinaire, des ganglions lymphatiques, du sein, en présence de gliomes, d'arthrite et d'asthme,
- destiné à une thérapie génétique locale,
- à une thérapie intra-artérielle de métastases du foie,
- pour traiter les maladies neurodégénérescentes et autoimmunitaires,
- en présence de la maladie de Parkinson, d'Alzheimer et de la sclérose en plaques,
- en présence du diabète de type I,
- en accompagnement de transplantations,
- pour traiter la resténose et/ou
- l'hypertension.
